(19) Europäisches Patentamt
European Patent Office
Office européen des brevets

(11) EP 0 878 185 A2

(12) **EUROPEAN PATENT APPLICATION**

(43) Date of publication:
**18.11.1998 Bulletin 1998/47**

(51) Int. Cl.[6]: **A61K 7/06**

(21) Application number: **98108719.0**

(22) Date of filing: **13.05.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **16.05.1997 JP 127366/97**

(71) Applicants:
- **SHOWA DENKO KABUSHIKI KAISHA**
  **Minato-ku, Tokyo (JP)**
- **Kosé Corporation**
  **Chuo-ku, Tokyo 103-0027 (JP)**

(72) Inventors:
- **Miyagawa, Satsuki**
  **Tokyo, 114-0005 (JP)**
- **Hinata, Takehiko**
  **Minato-ku, Tokyo, 105-8518 (JP)**
- **Yamaguchi, Tetsuhiko,**
  **c/o Showa Denko K.K.**
  **Kawasaki-shi, Kanagawa, 210-0867 (JP)**

(74) Representative:
**Strehl Schübel-Hopf & Partner**
**Maximilianstrasse 54**
**80538 München (DE)**

# (54) Hair-care preparations

(57) Disclosed is a hair-care preparation containing a homopolymer or copolymer comprising a repeating unit which is derived from an N-vinylcarboxamide monomer represented by general formula (1)

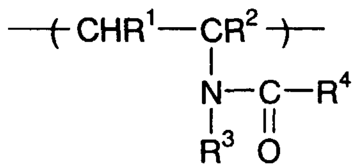

(1)

wherein $R^1$ and $R^2$ independently are a hydrogen atom, a methyl group or an ethyl group, $R^3$ and $R^4$ independently are a hydrogen atom or a methyl group, or a copolymer of the repeating unit represented by the general formula (1) and one or more other repeating units. The hair-care preparation has setting retaining power, gives satisfactory hair touch and feeling during and after use, and exhibits good resistance to moisture and good detergency upon shampooing.

EP 0 878 185 A2

## Description

### Background of the Invention

### Field of the Invention

This invention relates to hair-care preparations and more particularly to hair cosmetics which retains hair settings and gives satisfactory hair touch, use feeling during and after use and which contains a polymer comprising a repeating unit derived from a monomer containing an N-vinylcarboxamide as a major component that has high resistance to moisture and good detergency during shampooing.

### Description of Related Art

In hair cosmetics, various polymers such as nonionic, cationic, anionic and amphoteric polymers have heretofore been used as a hair setting polymer. Also, in order to give hairs conditioning effects or to increase holding ability and usability, there have been proposed a combination of an anionic polymer and a cationic polymer (Japanese Patent Publication (Kokoku) No. 62-7164 corresponding to U.S. Patent 4,445,521) and a combination of an amphoteric polymer and a cationic polymer (Japanese Patent Publication (Kokoku) No. 3-14805 corresponding to U.S. Patent 4,996,059) as well as development of new polymers for hairdressing (Japanese Patent Publication (Kokoku) No. 62-32165 corresponding to U.S. Patent 4,358,567 and Japanese Patent Application Laid-open (Kokai) No. 4-193822).

### Summary of the Invention

However, although hair-care preparations that contain conventionally used hairdressing polymers are sufficient as a hair setting polymer for retaining hair setting, they have problems in that they give poor hair touch, they make hair sticky or stiff to the touch during or after use, they are less resistant to moisture or they are difficult by removed by shampoo when they are intended to improve resistance to moisture. As a polymer for conditioning or for thickening, they are less compatible with ionic substances or they tend to accumulate on hair to make it stiff, thus making it less smooth so that it is difficult to give satisfactory feeling upon use.

Under the circumstances, the present inventors have made intensive investigation in order to solve the above-described problems and as a result found that a polymer comprising as a repeating unit a monomer containing N-vinylcarboxamide as a major component has a good setting retaining property as a polymer for setting while it is a suitable polymer for hair-care preparation which gives excellent feeling upon use as a conditioning polymer. This invention has been completed based on this discovery. That is, this invention provides the following hair-care preparations.

[1] A hair-care preparation comprising a polymer comprising a repeating unit represented by general formula (1)

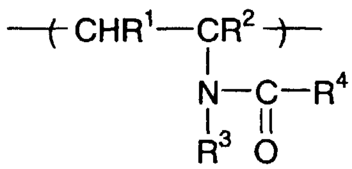

(1)

wherein $R^1$ and $R^2$ independently are a hydrogen atom, a methyl group, or an ethyl group; $R^3$ and $R^4$ independently are a hydrogen atom or a methyl group.

[2] A hair-care preparation as described in [1], wherein the polymer comprises a homopolymer of the repeating unit represented by general formula (1) as described in [1], or copolymer of said repeating unit as a major component.

[3] The hair-care preparation as described in [1], wherein the polymer comprises a copolymer of 10 to 99.9% by weight of the repeating unit represented by the general formula (1) as described in [1] and 0.1 to 90% by weight of at least one of repeating units represented by general formulae (2) to (6) and quaternary ammonium salts thereof:

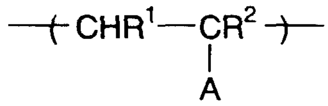

(2)

wherein $R^1$ and $R^2$ have the same meanings as defined above; A is a straight- or branched hydroxyalkyl group having 1 to 4 carbon atoms or a -Ph-$R^5$ group (where $R^5$ is a hydrogen atom or straight- or branched-chain alkyl group having 1 to 4 carbon atoms), a -COOR$^6$ group (where $R^6$ is a straight- or branched-chain alkyl, alkenyl or hydroxyalkyl group having 1 to 24 carbon atoms), a -OR$^7$ group (where $R^7$ is a straight- or branched-chain alkyl group having 1 to 4 carbon atoms), a -OCOR$^8$ group (where $R^8$ is a straight- or branched-chain alkyl group having 1 to 10 carbon atoms), a $-COO(C_2H_4O)_a(C_3H_6O)_bR^9$ group (where $R^9$ is a hydrogen atom or a straight- or branched-chain alkyl group having 1 to 4 carbon atoms, a and b are 0 or integers of 1 to 30, except for a=b=0 ), a -CONR$^{10}$R$^{11}$ group (where $R^{10}$ and $R^{11}$ independently are a hydrogen atom or a straight- or branched-chain alkyl group having 1 to 4 carbon atoms), or a group

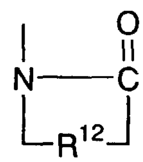

(wherein $R^{12}$ is an alkylene group having 3 to 7 carbon atoms);

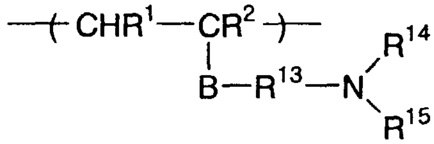

(3)

wherein $R^1$ and $R^2$ have the same meanings as defined above, B is an ether, ester or amide bonding, $R^{13}$ is an alkylene or hydroxyalkylene group having 1 to 4 carbon atoms, $R^{14}$ and $R^{15}$ independently are a methyl group or an ethyl group;

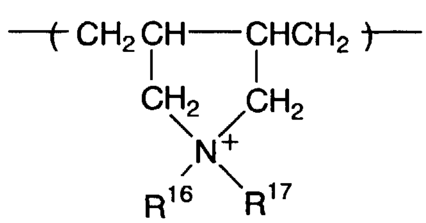

or

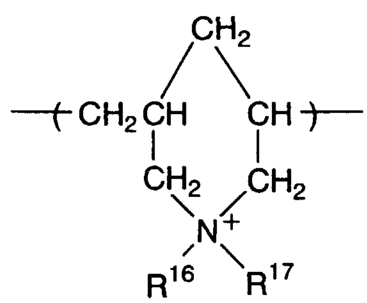

(4)

wherein $R^{16}$ and $R^{17}$ independently are a straight- or branched-chain alkyl group having 1 to 4 carbon atoms;

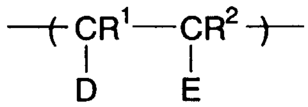

(5)

wherein $R^1$ and $R^2$ have the same meanings as defined above, D and E independently are a $-R^{18}COOM$, $-R^{18}SO_3M$ or $-R^{18}OPO_3M_2$ group (where M is a hydrogen atom or an alkali metal, $R^{18}$ is a simple bonding or an alkylene or hydroxyalkylene group having 1 to 4 carbon atoms), provided that when D and E are $-R^{18}COOM$ simultaneously, they may combine to form a cyclic anhydride group); or

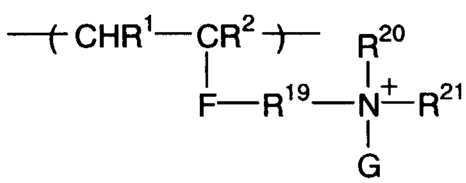

(6)

wherein $R^1$ and $R^2$ have the same meanings as defined above, F is an ether, ester or amide bonding, $R^{19}$ is an alkylene or hydroxyalkylene group having 1 to 4 carbon atoms, $R^{20}$ and $R^{21}$ independently are a methyl group or an ethyl group, G is a $-R^{22}COOM$, $-R^{22}SO_3M$ or $-R^{22}OPO_3M_2$ group (where M has the same meaning as defined above, $R^{22}$ is a simple bonding or an alkylene or hydroxyalkylene group having 1 to 4 carbon atoms).

[4] The hair-care preparation as described in [3], wherein the polymer comprises a copolymer of 20 to 99.9% by weight of the repeating unit represented by general formula (1) described in [1] and 0.1 to 80% by weight of at least one of repeating units represented by general formula (7) and quaternary ammonium salts thereof :

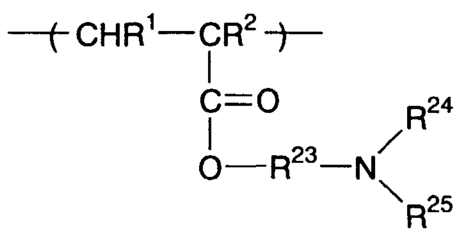

(7)

wherein $R^1$ and $R^2$ have the same meanings as defined above, $R^{23}$ is an alkylene or hydroxyalkylene group having 1 to 4 carbon atoms, $R^{24}$ and $R^{25}$ independently are a methyl group or an ethyl group.

[5] The hair-care preparation as described in [3], wherein the polymer comprises a copolymer of 20 to 99.9% by weight of the repeating unit represented by general formula (1) described in [1] and 0.1 to 80% by weight of at least one of repeating units represented by general formula (8) and quaternary ammonium salts thereof :

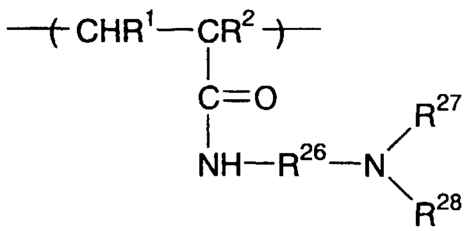

(8)

wherein $R^1$ and $R^2$ have the same meanings as defined above, $R^{26}$ is an alkylene or hydroxyalkylene group having 1 to 4 carbon atoms, $R^{27}$ and $R^{28}$ independently are a methyl group or an ethyl group.

[6] The hair-care preparation as described in any of [1] to [5], wherein the polymer has a viscosity in 30% ethanol solution at 20°C as measured by Brookfield viscometer of 50 to 10,000 cps.

[7] The hair-care preparation as described in any of [1] to [6], wherein the content of the polymer or copolymer is 0.01 to 40% by weight of the total composition.

Hereafter, the hair-care preparations of this invention will be described in detail.

The polymer comprising a repeating unit, represented by general formula (1) above, which is derived from an N-vinylcarboxamide monomer is a nonionic polymer which is soluble in water and at the same time is alcohol-philic property. The polymer exhibits good compatibility with deionized water and glycol as well as ionic substances. Further, the polymer shows no spinnability that is characteristic of viscous substances and its film gives no sticky nor stiff touch and it shows a good setting retaining property and is excellent in detergency when shampooing.

The repeating unit represented by general formula (1) is derived from an N-vinylcarboxamide . The N-vinylcarboxamide includes N-vinylacetamide, N-vinylformamide, N-methyl-N-vinylformamide, N-methyl-N-vinylacetamide and etc. Of these, preferred is N-vinylacetamide.

A monomer, for the repeating unit represented by general formula (2), which may be copolymerized with the N-vinylcarboxamide monomer for repeating unit represented by the general formula (1) above includes allyl alcohol, methallyl alcohol, etc., when A is a straight- or branched-chain hydroxyalkyl group having 1 to 4 carbon atoms; aromatic and unsaturated monomers such as styrene, allylbenzene, vinyltoluene, etc. when A is a -Ph-$R^5$ group (where $R^5$ is a hydrogen atom or straight- or branched-chain alkyl group having 1 to 4 carbon atoms); methyl (meth)acrylate, isobutyl (meth)acrylate, cyclohexyl (meth)acrylate, octyl (meth)acrylate, lauryl (meth)acrylate, oleyl (meth)acrylate, behenyl (meth)acrylate, hydroxyethyl (meth)acrylate, hydroxypropyl (meth)acrylate, etc. when A is a -$COOR^6$ group (where $R^6$ is a straight- or branched-chain alkyl, alkenyl or hydroxyalkyl group having 1 to 24 carbon atoms); methyl vinyl ether, ethyl vinyl ether, propyl vinyl ether, etc. when A is a -$OR^7$ group (where $R^7$ is a straight- or branched-chain alkyl group having 1 to 4 carbon atoms); vinyl ester monomers such as vinyl acetate, vinyl propionate, and vinyl neodecanoate when A is a -$OCOR^8$ (where $R^8$ is a straight- or branched-chain alkyl group having 1 to 10 carbon atoms); polyethylene glycol mono (meth)acrylate, methoxypolyethylene glycol mono(meth)acrylate, methoxypoly(ethylene glycol/propylene glycol) mono(meth)acrylate, etc. when A is a -$COO(C_2H_4O)_a(C_3H_6O)_bR^9$ (where $R^9$ is a hydrogen atom, or a straight- or branched-chain alkyl group having 1 to 4 carbon atoms, a and b are 0 or an integer of 1 to 30 except for $a=b=0$ ); acrylamide, N-t-butylacrylamide, isopropylacrylamide, dimethylacrylamide, etc. when A is a -$CONR^{10}R^{11}$ (where $R^{10}$ and $R^{11}$ independently are a hydrogen atom, or a straight- or branched-chain alkyl group having 1 to 4 carbon atoms); and N-containing cyclic vinyls such as N-vinylpyrolidone and N-vinylcaprolactam when A is represented by general formula (A) below

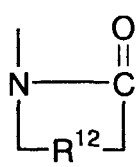

(A)

(where $R^{12}$ is an alkylene group having 3 to 7 carbon atoms).

The repeating unit represented by general formula (3) include repeating units represented by general formula (7) below

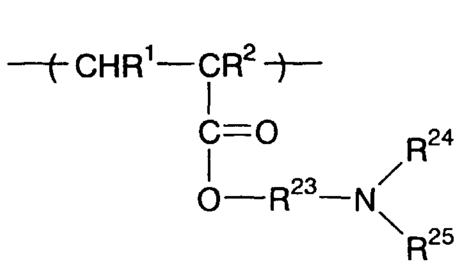

(7)

(where $R^1$ and $R^2$ have the same meanings as above, $R^{23}$ is an alkylene or hydroxyalkylene group having 1 to 4 carbon atoms, and $R^{24}$ and $R^{25}$ independently are a methyl group or an ethyl group) and repeating units represented by general formula (8)

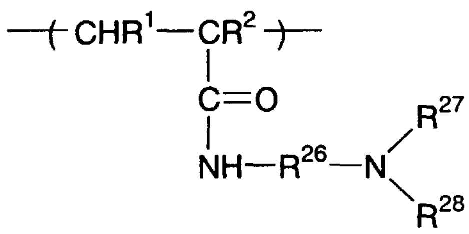

(8)

(where $R^1$ and $R^2$ have the same meanings as above, $R^{26}$ is an alkylene or hydroxyalkylene group having 1 to 4 carbon atoms, and $R^{27}$ and $R^{28}$ independently are a methyl group or an ethyl group).

Specific examples thereof include dimethylaminoethyl (meth)acrylate, diethylaminoethyl (meth)acrylate, dimethylaminopropyl (meth)acrylate, dimethylaminobutyl (meth)acrylate, dimethylaminoethyl (meth)acrylamide, dimethylaminopropyl (meth)acrylamide, etc. and their neutralization products neutralized with acids such as hydrochloric acid and lactic acid; their modified products modified with alkyl halide such as methyl chloride, ethyl chloride, methyl bromide, and ethyl iodide; their modified products modified with halogenated fatty acid esters such as ethyl monochloroacetate and methyl monochloropropionate; their modified product modified with dialkylsulfuric acids such as dimethylsulfuric acid and diethylsulfuric acid.

Also, there can be cited as specific examples, epihalohydrin quaternarized products between (meth)acrylic acid and trialkylamine, such as (meth)acryloyloxyhydroxypropyltrimethylammonium chloride, (meth)acryloyloxyhydroxypropyltriethylammonium chloride and (meth)acryloyloxyhydroxypropyltriethylammonium bromide.

These cationic repeating units can be introduced in the polymer by copolymerizing the above-described monomers or by an alternative method in which its precursor is copolymerized and the polymer is converted to a cation with a modifying agent.

More specifically, a method is exemplified in which polymerization is performed using dimethylaminoethyl (meth)acrylate, which is a precursor, and then the resulting polymer is modified with a modifying agent (such as hydrochloric acid, ethyl monochloroacetate, dimethylsulfuric acid, etc.) to form a cation.

A monomer for the repeating unit represented by general formula (4) includes diallyldimethylammonium chloride, diallyldiethylammonium chloride, etc.

A monomer for the repeating unit represented by general formula (5) includes unsaturated carboxylic acid-based monomers such as (meth)acrylic acid, maleic acid, maleic anhydride, itaconic acid, fumaric acid and crotonic acid; unsaturated dicarboxylic acid monoester-based monomers such as ethyl maleate and butyl maleate; half-esters between unsaturated polycarboxylic anhydrides (such as succinic anhydride and phthalic acid anhydride) and hydroxy group-containing (meth)acrylates (such as hydroxyethyl (meth)acrylate and hydroxypropyl (meth)acrylate); sulfonic acid group-containing monomers such as 2-acrylamide-2-methylpropanesulfonic acid, 2-(meth)acrylamideethanesulfonic acid, (meth)acrylic acid-methylsulfonic acid, (meth)acrylic acid-2-ethylsulfonic acid and (meth)acrylic acid-3-propylsulfonic acid; phosphoric acid group containing monomers such as allylphosphoric acid and acid phosphooxyethyl (meth)acrylate.

These anionic repeating units may be used as they are in the form of acid or after partial or complete neutralization. Alternatively, they may be copolymerized as they are in the form of acid and then partially or completely neutralized.

As a neutralizing agent, there can be used alkali metal hydroxides such as potassium hydroxide and sodium hydroxide; ammoniacal water; amine compounds such as mono- di- or triethanolamine, triethylamine, morpholine, aminomethylpropanol, and aminoethylpropanediol; and so on.

As the repeating unit represented by general formula (6), there can be cited amine derivatives of (meth)acrylic acid and amine derivatives of (meth)acrylamide, for example, dimethylaminoethyl (meth)acrylate and dimethylaminopropyl (meth)acrylamide, that are modified with monohalogenated fatty acid salts such as aminomethylpropanol salt of monochloroacetic acid, triethanolamine salt of monochloroacetic acid, potassium monochloroacetate, and lithium monochloropropionate, and those modified with propanesultone.

These amphoteric repeating units can be obtained by copolymerization of monomers or alternatively they can be obtained by copolymerizing precursors thereof followed by converting the resulting copolymers to amphoteric products with a modifying agent. As the modifying agent, there can be used, for example, monohalogenated fatty acid alkali metal salts, such as sodium monobromoacetate, sodium monochloroacetate, potassium monochloroacetate, and lithium monochloropropionate; monohalogenated fatty acids neutralized with ammonia, aminomethylpropanol, monoethanolamine, diethanolamine, triethanolamine, aminomethylpropanediol, aminoethylpropanol, morpholine or the like. When the amphoteric (amphiprotizing) agent is sodium, potassium, lithium or the like alkali metal salt, inorganic salts will be precipitated as the amphiprotization reaction proceeds so that it is preferred that the salts be removed by solid-liquid separating operation such as centrifugation and filtration if they are harmful upon blending. On the contrary, when the amphiprotizing agent is a amine salt or ammonium salt, no organic salt will be precipitated and, hence, it is unnecessary to conduct the above-described separating operation and the reaction mixture can be used as it is as a homogeneous solution.

The proportion of the monomer for the repeating units represented by the general formulae (2) to (6) which are copolymerized with the monomer for the repeating units represented by the general formula (1) is 0.1 to 90% by weight, preferably 1 to 85% by weight.

The monomer for the repeating unit which is copolymerized with the N-vinylcarboxamide monomer for the repeating units represented by general formula (1) is a component which imparts the film formed after drying with flexibility, adherability, luster and smoothness. If the proportion of this repeating unit is below the above-described range, the effects to be obtained by blending it is insufficient. On the other hand, if the proportion of the repeating unit exceeds the above-described upper limit, the characteristic features of the polymer represented by general formula (1), i.e., solubility in water, alcohol or ionic substances, film-forming properties and detergency, will be degraded.

Although no limitation is posed on the process of polymerizing monomers represented by general formula (1) nor the process of copolymerizing the monomers with other monomers copolymerizable therewith, usually it is preferred that such a polymerization or copolymerization reaction be carried out by a precipitation polymerization process, an aqueous solution polymerization process, an organic solvent polymerization process and the like.

For example, in the precipitation polymerization process and the organic solvent polymerization process, a monomer such as N-vinylacetamide is alone or together with a monomer copolymerizable therewith are reacted in an organic solvent such as benzene, toluene, xylene, ethylbenzene, acetone, methyl ethyl ketone, hexane, heptane, octane, ethyl acetate, butyl acetate, isopropyl acetate, methyl alcohol, ethyl alcohol, and isopropyl alcohol in the presence of a solvent-soluble polymerization initiator such as azobisisobutyronitrile, benzoyl peroxide, t-butyl hydroxyperoxide, or di-(2-ethylhexylperoxydicarbonate).

Further, as the aqueous solution polymerization process, there can be used a process in which a monomer component is uniformly dissolved in water or a mixed solvent containing water and a hydrophilic organic solvent which is uniformly miscible with water, such as methanol, tetrahydrofuran or acetone, and a polymerization initiator is added to copolymerize after removing the dissolved oxygen in the system by vacuum degasification or purging with an inert gas such as nitrogen or carbon dioxide gas.

As the polymerization initiator, there can be used peroxides, organic or inorganic peracids or salts thereof, azobis-based compounds, alone or as redox systems in combination with reducing agents. Representative examples thereof include t-butyl peroxide, benzoyl peroxide, t-butyl hydroxyperoxide, azobisisobutyronitrile, 2,2'-azobis(2-amidinopropane) dihydrochloride, 2,2'-azobis[2-(5-methyl-2-imidazoline-2-yl)propane] dihydrochloride, sodiumpersulfate, ammoniumpersulfate, hydrogen peroxide water, and combinations of persulfates with tertiary amines such as triethylamine and triethanolamine.

These polymers can be employed as they are dissolved in the hydrophilic solvent used in the reaction, or diluted with water, ethanol or subjected to solvent replacement with water, if desired. These polymers can be separated as polymer solids by removing the solvent from the solution. The thus obtained polymer solids may be further dilluted with a solvent and may be used as a polymer solution of copolymer. Two or more polymers and/or solutions thereof may be mixed for use.

It is preferred that these polymers have a solution viscosity in 30% ethanol solution at 20°C by Brookfield viscometer is in the ranges of 50 to 10,000 cps, more preferably in the ranges of 100 to 5,000 cps. If the solution viscosity is below 50 cps, the polymer is flexible or brittle and is poor in hair holding ability as a hair holding polymer while it has insufficient viscosity as a thickening polymer, so that such polymer is difficult to put into practical use. On the other hand, if the solution viscosity exceeds 10,000 cps, the polymer shows a considerable decrease in solubility or a considerable increase in stringiness, so that when it is blended in hair-care preparations, it causes the preparations to become clammy or sticky upon use, thus giving unpleasant touch.

These polymers can be used as a component to be blended in hair-care preparations in general, for example, styling agents such as mousse, styling gel and hair spray, shampoo, rinse, treatments and the like.

The amount of the above-described polymer to be blended in hair-care preparations is desirably in the ranges of 0.01 to 40% by weight as net polymer. If this blending amount is below 0.01% by weight, the effects expected will be obtained only insufficiently by the addition of the polymer while if the amount exceeds 40% by weight, hair will become clammy or stiff, thus giving considerably deteriorated hair touch.

The hair-care preparations of this invention may contain, in addition to the above polymer, other components which are normally blended in a hair-care preparation such as viscosity adjusting agent, film-forming agent, hair-conditioning agent, pH adjusting agent, detergent, emulsifying agent, emulsifying aid and propellant.

As a viscosity adjusting agent, there can be used water-soluble polymers such as methylcellulose, carboxymethylcellulose, hydroxyethylcellulose, and xanthane gum and nonionic surfactants such as fatty acid alkylolamides.

As a film-forming agent, there can be used cationic polymers such as cationized cellulose, cationized starch, cationized guar gum, vinylpyrrolidone, N,N-dimethylaminoethyl methacrylic acid copolymer quaternarized salt product, and diallyl quaternary ammonium salt polymers; nonionic polymers such as polyvinylpyrrolidone, polyvinylpyrrolidone/vinyl acetate copolymers, and polyvinyl alcohol; anionic polymers such as methyl vinyl ether/maleic acid half ester copolymers, acrylic resin alkanolamine solution; amphoteric copolymers such as N-methacryloyloxyethylene, N,N-dimethylammonium-$\alpha$-N-methylcarboxybetain methacrylic acid alkyl ester copolymers, and the like.

As a hair-conditioning agent, there can be used silicone derivatives such as low viscosity silicone, highly polymerized silicone, cyclic silicone, polyether-modified silicone, amino-modified silicone, cationic silicone or cationic surfactants and etc.

As pH adjusting agents, there can be used acids such as citric acid and lactic acid or salts thereof. As the detergents, there can be used various anionic and amphoteric surfactants known in the art. As an emulsifying agent, there can be cited nonionic surfactants such as polyoxyalkylene-adduct type surfactants. As an emulsifying aid, there can be used higher alcohols and glycerol fatty acid esters. Propellants which can be used include liquefied petroleum gas, nitrogen gas, carbon dioxide, dimethyl ether, and the like.

In addition thereto, there can be added various other components which are blended usually in cosmetic preparations, for example, higher fatty acids, straight- or branched-chain esters, hydrocarbons, oily components such as oils and fats, hydrophilic components such as polyhydric alcohols and lower alcohols, perfumes, preservatives, UV absorbents, antioxidants, sterilizers, pharmaceutical ingredients, and the like.

The hair-care preparations of this invention can be applied in various forms such as liquid, emulsion, cream, gel, mousse, mist, and the like in combination with other ingredients or utilizing the mechanism of a vessel.

## Effects of the Invention

As described in detail above, the hair-care preparations of this invention comprising the polymer comprising the repeating unit represented by the general formula (1) above, polymer comprising the repeating unit represented by the general formula (1) and at least one of the repeating units represented by the general formulae (2) to (6) above and their quaternary salts well retain hair setting, show as a hair setting agent substantially no sticky or stiff touch during or after use and give flexible and smooth touch to hair, and as a conditioning agent allow fingers through hair smoothly, thus excellent combing and hair dressing effects as compared with the conventional hair-care preparations.

## Best Modes for Carrying Out the Invention

Hereafter, this invention will be described in more detail by synthetic examples, comparative synthetic examples, examples and comparative examples. However, this invention should not be construed as being limited thereto.

### Synthesis Example 1

In a four-necked flask equipped with a reflux condenser, a thermometer, a dropping funnel, a glass tube for nitrogen purging, and a stirrer were charged 50 g of N-vinylacetamide and 450 g of benzene. After introducing nitrogen gas thereto to establish a sufficient nitrogen atmosphere, 0.2 g of azobisisobutyronitrile was added and the mixture was heated for 3 hours under reflux for polymerization. The polymer which precipitated was filtered and dried in vacuum to obtain 47 g of polymer solids.

### Synthesis Example 2

The same procedures as Synthesis Example 1 above were repeated except that the N-vinylacetamide was replaced by 35 g of N-vinylacetamide and 15 g of 2-hydroxypropyl methacrylate and the benzene was replaced by 450 g of ethyl acetate to obtain 48.7 g of polymer solids.

### Synthesis Example 3

The same procedures as Synthesis Example 1 were repeated except that the N-vinylacetamide was replaced by 12 g of N-vinylacetamide and 38 g of N-vinylpyrolidone, the benzene was replaced by 450 g of ethyl acetate, and 0.4 g of azobisisobutyronitrile was used to obtain 42.5 g of polymer solids.

### Synthesis Example 4

The same procedures as in Synthesis Example 1 were repeated except that the N-vinylacetamide was replaced by 35 g of N-vinylformamide and 15 g of methyl acrylate, the benzene was replaced by 450 g of toluene, and the azobisisobutyronitrile as a polymerization initiator was replaced by 0.2 g of benzoyl peroxide to obtain 46.3 g of polymer solids.

Synthesis Example 5

The same procedures as in Synthesis Example 1 were repeated except that the N-vinylacetamide was replaced by 25 g of N-vinylacetamide and 25 g of vinyl acetate, the benzene was replaced by 450 g of ethyl acetate, and the azobisisobutyronitrile as a polymerization initiator was replaced by 0.2 g of benzoyl peroxide to obtain 40.0 g of polymer solids.

Synthesis Example 6

In a four-necked flask equipped with a reflux condenser, a thermometer, a dropping funnel, a glass tube for nitrogen purging, and a stirrer were charged 40 g of N-vinylacetamide, 10 g of N, N-dimethylaminoethyl methacrylate methyl chloride salt, and 450 g of methanol. After introducing nitrogen gas thereto to establish a sufficient nitrogen atmosphere, 0.5 g of azobisisobutyronitrile was added and the mixture was heated for 3 hours under reflux for polymerization. After distilling off the solvent, the reaction mixture was dried in vacuum to obtain 49.5 g of polymer solids.

Synthesis Example 7

In a four-necked flask equipped with a reflux condenser, a thermometer, a dropping funnel, a glass tube for nitrogen purging, and a stirrer were charged 33 g of N-vinylacetamide, 17 g of N, N-dimethylaminoethyl methacrylate methyl chloride salt, and 450 g of deionized water. After introducing nitrogen gas thereto to sufficiently expel oxygen, the water temperature was adjusted to 30°C and 1 g of 2,2'-azobis(2-amidinopropane) dihydrochloride was added and the mixture was allowed to polymerize for 6 hours. The 10% aqueous polymer solution as it was used for preparing a composition.

Synthesis Example 8

The same procedures as Synthesis Example 1 above were repeated except that the N-vinylacetamide was replaced by 34.5 g of N-vinylacetamide and 15.5 g of N, N-dimethylaminoethyl methacrylate, the benzene was replaced by 450 g of ethyl acetate, and 0.5 g of azobisisobutyronitrile as a polymerization initiator to obtain 48.0 g of polymer solids.

Synthesis Example 9

The same procedures as Synthesis Example 1 were repeated except that the N-vinylacetamide was replaced by 27 g of N-vinylacetamide and 10 g of vinyl acetate, and 13 g of dimethylaminopropylacrylamide, the benzene was replaced by 450 g of ethyl acetate, and 0.5 g of azobisisobutyronitrile as a polymerization initiator was used to obtain 41.4 g of polymer solids.

Synthesis Example 10

The same procedures as in Synthesis Example 1 were repeated except that the N-vinylacetamide was replaced by 27 g of N-vinylacetamide and 23 g of acrylic acid, the benzene was replaced by 450 g of ethyl acetate, and 0.5 g of azobisisobutyronitrile as a polymerization initiator was used to obtain 45.5 g of polymer solids.

Synthesis Example 11

In a four-necked flask equipped with a reflux condenser, a thermometer, a dropping funnel, a glass tube for nitrogen purging, and a stirrer were charged 34.5 g of N-vinylacetamide, 15.5 g of N, N-dimethylaminoethyl methacrylate, and 450 g of ethanol. After introducing nitrogen gas thereto to establish a sufficient nitrogen atmosphere, 0.5 g of azobisisobutyronitrile was added and the mixture was heated under reflux to polymerize for 4 hours. After completion of the polymerization, 40% by weight ethanol suspension of 13.4 g of potassium monochloroacetate was dripped down from the dropping funnel, and the reaction mixture was heated under reflux for further 6 hours for amphiprotization under nitrogen stream. After completion of the reaction, the polymer solution was filtered under pressure to separate and remove inorganic salts. After distilling off the solvent, the reaction mixture was dried in vacuum to obtain 42.2 g of polymer solids.

Synthesis Example 12

In a four-necked flask equipped, with a reflux condenser, a thermometer, a dropping funnel, a glass tube for nitrogen purging, and a stirrer were charged 25 g of N-vinylacetamide, 15 g of N, N-dimethylaminoethyl methacrylate, 10 g of acrylic acid, and 450 g of ethanol. After introducing nitrogen gas thereto to establish a sufficient nitrogen atmosphere, 0.5 g of azobisisobutyronitrile was added and the mixture was heated under reflux to polymerize for 4 hours. After completion of the polymerization, 8.8 g of ethyl bromide was carefully dripped down from the dropping funnel at temperatures below its boiling point, and the reaction mixture was kept at the temperature at which ethyl bromide was added for 1 hour. Then, the mixture was heated under reflux for further 6 hours for quaternarization. After completion of the reaction, the polymer obtained was dried in vacuum after distilling off the solvent to obtain 46.3 g of polymer solids.

Synthesis Example 13

The same procedures as Synthesis Example 1 above were repeated except that the N-vinylacetamide was replaced by 7.5 g of N-vinylacetamide and 42.5 g of N-vinylpyrolidone, the benzene was replaced by 450 g of ethyl acetate, and 0.3 g of azobisisobutyronitrile as a polymerization initiator was used to obtain 49.5 g of polymer solids.

Synthesis Example 14

The same procedures as, Synthesis Example 1 above were repeated except that the N-vinylacetamide was replaced by 10 g of N-vinylacetamide and 40 g of N, N-dimethylaminoethylmethacrylate, the benzene was replaced by 450 g of ethyl acetate, and 0.3 g of azobisisobutyronitrile as a polymerization initiator was used to obtain 49 g of polymer solids.

Synthesis Example 15

The same procedures as Synthesis Example 1 above were repeated except that the N-vinylacetamide was replaced by 10 g of N-vinylacetamide and 40 g of N, N-dimethylaminopropylmethacrylamide, the benzene was replaced by 450 g of ethyl acetate, and 0.3 g of azobisisobutyronitrile as a polymerization initiator was used to obtain 47 g of polymer solids.

Synthesis Example 16

In a four-necked flask equipped with a reflux condenser, a thermometer, a dropping funnel, a glass tube for nitrogen purging, and a stirrer were charged 42 g of N-vinylacetamide, 18 g of N, N-dimethylaminoethylmethacrylamide, and 140 g of ethanol. After introducing nitrogen gas thereto to establish a sufficient nitrogen atmosphere, 0.6 g of azobisisobutyronitrile was added and the mixture was heated under reflux to polymerize for 8 hours. A 30% ethanol solution of the polymer obtained was used as it was for preparing a composition.

Synthesis Example 17

In a four-necked flask equipped with a reflux condenser, a thermometer, a dropping funnel, a glass tube for nitrogen purging, and a stirrer were charged 75 g of N-vinylacetamide, 20 g of N, N-dimethylaminopropylmethacrylamide, 5 g of n-butyl methacrylate, and 100 g of ethanol. After introducing nitrogen gas thereto to establish a sufficient nitrogen atmosphere, 0.5 g of azobisisobutyronitrile was added and the mixture was heated under reflux to polymerize for 8 hours. A 50% ethanol solution of the polymer obtained was used as it was for preparing a composition.

Comparative Synthesis Example 1

In a four-necked flask equipped with a reflux condenser, a thermometer, a dropping funnel, a glass tube for nitrogen purging, and a stirrer were charged 53 g of N-vinylacetamide, 15 g of N, N-dimethylaminopropylmethacrylamide, 3 g of n-butyl methacrylate, 1.5 g of pentaerythritol trially ether, and 630 g of ethyl acetate. After introducing nitrogen gas thereto to establish a sufficient nitrogen atmosphere, 0.35 g of azobisisobutyronitrile was added and the mixture was heated under reflux to polymerize for 5 hours. The polymer which precipitated in the solvent was filtered and dried in vacuum to obtain 63 g of polymer solids.

Examples 1 to 5 and Comparative Examples 1 to 3: Styling Mousse

Styling mousses were prepared with formulations (parts by weight) shown in Table 1 and by the following process and evaluations were made on setting retaining power, hair touch such as sticky or stiff touch, hair detergency, and flaking conditions using human hair wigs. Table 1 below shows the results.

(I) Preparation Method:

(i) Components (1) to (12) were uniformly mixed to obtain a composition (A).
(ii) Composition (A) and component (13) were filled in an aerosol can to obtain a styling mousse.

(II) Evaluation Method:

Human hair wigs were treated with ordinary type commercially available shampoo and rinse and dried. Then, 5 g of samples of Examples 1 to 5 and Comparative Examples 1 to 3 were treated on the hair wigs, respectively and dried. The dried hair wigs were evaluated on holding ability and hair touch (sticky or stiff touch) according to evaluation standards (a) and (b) below. Further, the hair wigs thus treated were combed 5 times and the flaking conditions were evaluated according to the standards (c) below. Finally, the hair wigs were washed with ordinary type commercially available shampoo and detergency was evaluated according to the standards (d) below.

(III) Evaluation Standards:

(1) Hair holding ability

After setting human hair wigs, the holding ability and its durability were observed with the eyes.

Evaluation Standards (a)

A: Very good holding ability, and highly durable;
B: Holding ability is fairly strong and durable;
C: Holding ability is slightly weak and slightly less durable;
D: Holding ability is very weak and not so durable.

(2) Hair Touch (Absence of Sticky or Stiff Touch)

Evaluation Standards (b)

A: Substantially no sticky or stiff touch;
B: Slight sticky or stiff touch;
C: Sticky or stiff touch;
D: Very sticky or stiff touch.

(3) Degree of Flaking (Polymer Peeling)

After repeating combing 5 times, flaking conditions of the hair were observed with the eyes.

Evaluation Standards (c)

A: No flaking;
B: Slight flaking;
C: Flaking;
D: Considerable flaking.

(4) Detergency (Absence of creaking upon shampooing, whether or not removal of the polymer is easy)

Evaluation was made after shampooing with ordinary type commercially available shampoo.

Evaluation Standards (d)

A: Very good;
B: Good;
C: Poor;
D: Very poor.

Table 1

| Styling Mousse | | | | | | | | | |
|---|---|---|---|---|---|---|---|---|---|
| | | Ex.1 | Ex.2 | Ex.3 | Ex.4 | Ex.5 | C.Ex.1 | C.Ex.2 | C.Ex.3 |
| (1) | Polymer of Syn.Ex.1 | 3 | | | | | | | |
| (2) | Polymer of Syn.Ex.2 | | 5 | | | | | | |
| (3) | Polymer of Syn.Ex.3 | | | 3 | | | | | |
| (4) | Polymer of Syn.Ex.4 | | | | 5 | | | | |
| (5) | Polymer of Syn.Ex.5 | | | | | 5 | | | |
| (6) | PVA.VA | | | | | | 3 | | |
| (7) | PVP.DMAEM | | | | | | | 5 | |
| (8) | PMVE.MB | | | | | | | | 5 |
| (9) | Aminomethylpropanol | | | | | | | | 1 |
| (10) | Perfume | s.a. | s.a. | s.a. | s.a. | s.a. | s.a. | s.a. | s.a. |
| (11) | Ethanol | 10 | 10 | 10 | 10 | 10 | 10 | 10 | 10 |
| (12) | Deionized water | res. | res. | res. | res. | res. | res. | res. | res. |
| (13) | Propellant (Liquefied petroleum gas) | 3 | 3 | 3 | 3 | 3 | 3 | 3 | 3 |
| Setting retaining power | | A | A | B | A | A | C | A | D |
| Hair touch (absence of sticky or stiff touch) | | A | A | A | A | A | C | D | D |
| Degree of flaking | | A | A | A | A | A | D | C | A |
| Detergency | | A | A | A | A | A | C | D | B |

PVP.VA: Polyvinylpyrolidone/vinyl acetate copolymer
PVP.DMAEM: Vinylpyrolidone/N,N-dimethylaminoethyl methacrylate copolymer diethylsulfate
PMVE.MB: Methyl vinyl ether/butyl maleate copolymer
s.a.: suitable amount
res.: residual amount to make the total 100%

As evidenced from the results shown in Table 1, the styling mousses containing the polymers of this invention exhibited very excellent setting retaining power, hair touch, flaking conditions and detergency over those of Comparative Examples 1 to 3.

Examples 6 to 11 and Comparative Examples 4 to 7: Hair styling gel

Hair styling gels were prepared with formulations (% by weight) shown in Table 2 and by the method described below and evaluations were made on setting retaining power, hair touch (absence of sticky or stiff touch), degree of flaking and hair detergency (absence of creaking, conditions of polymer removal) using human hair wigs. Table 2 below shows the results.

Preparation Method:

(i) Components (1) to (16) were uniformly mixed to obtain a composition (A).

(ii) Composition (A) and component (17) were uniformly mixed to obtain a hair styling gel.

Table 2 Hair Styling Gel

| | Ex.6 | Ex.7 | Ex.8 | Ex.9 | Ex.10 | Ex.11 | C.Ex.4 | C.Ex.5 | C.Ex.6 | C.Ex.7 |
|---|---|---|---|---|---|---|---|---|---|---|
| (1) Polymer of Syn.Ex.1 | 7 | | | | | | | | | |
| (2) Polymer of Syn.Ex.6 | | 10 | | | | | | | | |
| (3) Polymer of Syn.Ex.8 | | | 10 | | | | | | | |
| (4) Polymer of Syn.Ex.9 | | | | 10 | | | | | | |
| (5) Polymer of Syn.Ex.10 | | | | | 8 | | | | | |
| (6) Polymer of Syn.Ex.17 | | | | | | 20 | | | | |
| (7) Polymer of C.Syn.Ex.1 | | | | | | | | | | 10 |
| (8) PVP.VA | | | | | | | 5 | | | |
| (9) PVP.DMAEM | | | | | | | 2 | | | |
| (10) AR.AA | | | | | | | | 10 | | |
| (11) AOA.AE | | | | | | | | | 8 | |
| (12) Aminomethyl propanol | | | | | | | | 2 | 2 | |
| (13) Perfume | s.a. | s.a. | s.a. | s.a. | s.a. | s.a. | s.a. | s.a. | s.a. | s.a. |
| (14) Ethanol | 20 | 20 | 20 | 20 | 20 | 20 | 20 | 20 | 20 | 20 |
| (15) Propylene glycol | 5 | 5 | 5 | 5 | 5 | 5 | 5 | 5 | 5 | 5 |
| (16) Deionized water | res. | res. | res. | res. | res. | res. | res. | res. | res. | res. |
| (17) Methylcellulose | 0.5 | 0.5 | 0.5 | 0.5 | 0.5 | 0.5 | 0.5 | 0.5 | 0.5 | 0.5 |

PVP.VA: Polyvinylpyrolidone/vinyl acetate copolymer
PVP.DMAEM: Vinylpyrolidone/N,N-dimethylaminoethyl methacrylate copolymer diethylsulfate
AR.AA: Acrylic resin alkanolamine
AOA.AE: Acrylic acid octylamide/acrylic acid ester copolymer
s.a.: suitable amount
res.: residual amount to make the total 100%

Table 2 Hair Styling Gel (Continued)

| | Ex.6 | Ex.7 | Ex.8 | Ex.9 | Ex.10 | Ex.11 | C.Ex.4 | C.Ex.5 | C.Ex.6 | C.Ex.7 |
|---|---|---|---|---|---|---|---|---|---|---|
| Hair setting retaining power | A | A | A | A | A | A | A | A | A | D |
| Hair touch (absence of sticky or stiff touch) | A | A | A | A | A | A | C | D | D | D |
| Degree of flaking | B | A | A | A | A | A | D | D | D | A |
| Detergency | A | A | A | A | A | A | D | D | D | B |

As evidenced from the results shown in Table 2, the hair styling gel containing the polymers of this invention exhibited very excellent setting retaining power, hair touch, flaking conditions and detergency over those of Comparative Examples 4 to 7.

Example 12: Shampoo

Table 3

| Formulation of Shampoo | | |
|---|---|---|
| Component | | Amount(wt%) |
| (1) | Sodium laurylsulfate | 8.0 |
| (2) | Triethanolamine laurylsulfate | 5.0 |
| (3) | Cocoyl amide propyldimethyl glycine | 5.0 |
| (4) | Diethylen glycol distearate | 2.0 |
| (5) | Propylene glycol | 10.0 |
| (6) | Polymer of Synthesis Example 12 | 1.0 |
| (7) | Perfume | s.a.[1)] |
| (8) | Preservatives | s.a.[1)] |
| (9) | Deionized water | res.[2)] |

1) Suitable amount
2) Residual amount to make the total 100%

In the above formulation, components (1) to (8) were added in sequence to component (9) and heated and mixed, followed by cooling.

Example 13: Shampoo

Shampoo was prepared in the same manner as in Example 12 except that the polymer of Synthesis Example 12 was replaced by the polymer of Synthesis Example 13. The shampoos of Examples of 12 and 13 allowed fingers through the hair smoothly during shampooing and rinsing and gave hair excellent combing and hair dressing properties after drying.

Example 14: Hair Rinse

Table 4

| Formulation of Hair Rinse | | |
|---|---|---|
| Component | | Amount(wt%) |
| (1) | Stearyl trimethylammonium chloride | 2.0 |
| (2) | Cetanol | 3.0 |
| (3) | Behenyl alcohol | 1.0 |
| (4) | Methylphenyl polysiloxane | 5.0 |
| (5) | Isopropyl myristate | 5.0 |
| (6) | Polyoxyethylene hydrogenated castor oil(20E.O.) | 1.0 |
| (7) | Propylene glycol | 10.0 |
| (8) | 10% Aqueous solution of Polymer of Synthesis Example 7 | 8.0 |
| (9) | Preservatives | s.a.[1)] |
| (10) | Perfume | s.a.[1)] |
| (11) | Deionized water | res.[2)] |

1) Suitable amount
2) Residual amount to make the total 100%

In the above formulation, components (1) and (6) to (10) were mixed with component (11) with heating, and on the other hand, components (2) to (4) were mixed with component (5) with heating. The both were mixed and emulsified to prepare hair rinse.

Example 15: Hair Rinse

Hair rinse was prepared in the same manner as in Example 14 except that the aqueous solution of the polymer of Synthesis Example 7 was replaced by 10% aqueous solution of the polymer of Synthesis Example 14. The hair rinses of Examples 13 and 14 allowed fingers through hair upon use and rinsing and gave hair excellent combing and hair dressing properties.

Example 16: Hair Cream

Table 5

| Formulation of Hair Cream | | |
|---|---|---|
| Component | | Amount(wt%) |
| (1) | Stearyl trimethylammonium chloride | 0.3 |
| (2) | Cetanol | 1.5 |
| (3) | Behenyl alcohol | 0.5 |
| (4) | Octyldodecyl myristate | 2.0 |
| (5) | Sorbitan monostearate | 0.1 |
| (6) | Polyoxyethylene hydrogenated castor oil(40E.O.) | 0.5 |
| (7) | Glycerol | 2.0 |
| (8) | Ethanol | 5.0 |
| (9) | Polymer of Synthesis Example 11 | 1.0 |
| (10) | Antiseptics | s.a.[1] |
| (11) | Perfume | s.a.[1] |
| (12) | Deionized water to make | res.[2] |

1)Suitable amount
2)Residual amount to make the total 100%.

In the above formulation, components (1) and (6) to (11) were mixed with component (12) with heating, and on the other hand, components (2) to (5) were mixed with heating. The both were mixed and emulsified to prepare hair cream.

Example 17: Hair Cream

Hair cream was prepared in the same manner as in Example 16 except that the polymer of Synthesis Example 11 was replaced by the polymer of Synthesis Example 15.

Example 18: Hair Cream

Hair cream was prepared in the same manner as in Example 16 except that the polymer of Synthesis Example 11 was replaced by a 30% ethanol solution of the polymer of Synthesis Example 16.

The hair creams of Examples 16 to 18 were not sticky upon use, and gave hair excellent hair touch and hair dressing properties.

**Claims**

**1.** A hair-care preparation comprising a polymer comprising a repeating unit represented by general formula (1)

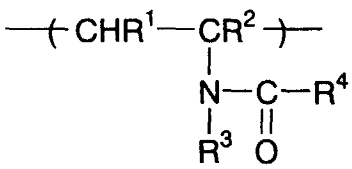

( 1 )

wherein $R^1$ and $R^2$ independently are a hydrogen atom, a methyl group, or an ethyl group; $R^3$ and $R^4$ independently are a hydrogen atom or a methyl group.

2. A hair-care preparation claimed in claim 1, wherein the polymer comprises a homopolymer of the repeating unit represented by general formula (1) described in claim 1, or copolymer of said repeating unit as a major component.

3. The hair-care preparation as claimed in claim 1, wherein the polymer comprises a copolymer of 10 to 99.9% by weight of the repeating unit represented by the general formula (1) described in claim 1 and 0.1 to 90% by weight of at least one of repeating units represented by general formulae (2) to (6) and quaternary ammonium salts thereof :

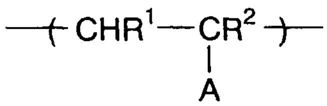

(2)

wherein $R^1$ and $R^2$ have the same meanings as defined above; A is a straight- or branched hydroxyalkyl group having 1 to 4 carbon atoms or a -Ph-$R^5$ group (where $R^5$ is a hydrogen atom or straight- or branched-chain alkyl group having 1 to 4 carbon atoms), a -$COOR^6$ group (where $R^6$ is a straight- or branched-chain alkyl, alkenyl or hydroxyalkyl group having 1 to 24 carbon atoms), a -$OR^7$ group (where $R^7$ is a straight- or branched-chain alkyl group having 1 to 4 carbon atoms), a -$OCOR^8$ group (where $R^8$ is a straight- or branched-chain alkyl group having 1 to 10 carbon atoms), a -$COO(C_2H_4O)_a(C_3H_6O)_bR^9$ group (where $R^9$ is a hydrogen atom or a straight- or branched-chain alkyl group having 1 to 4 carbon atoms, a and b are 0 or integers of 1 to 30, except for a=b=0 ), a -$CONR^{10}R^{11}$ group (where $R^{10}$ and $R^{11}$ independently are a hydrogen atom or a straight- or branched-chain alkyl group having 1 to 4 carbon atoms), or a group

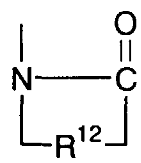

(wherein $R^{12}$ is an alkylene group having 3 to 7 carbon atoms);

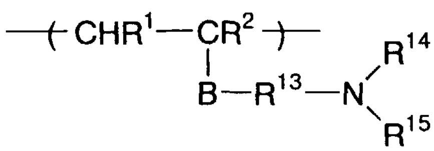

(3)

wherein $R^1$ and $R^2$ have the same meanings as defined above, B is an ether, ester or amide bonding, $R^{13}$ is an alkylene or hydroxyalkylene group having 1 to 4 carbon atoms, $R^{14}$ and $R^{15}$ independently are a methyl group or an ethyl group;

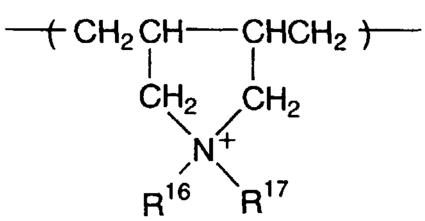

or

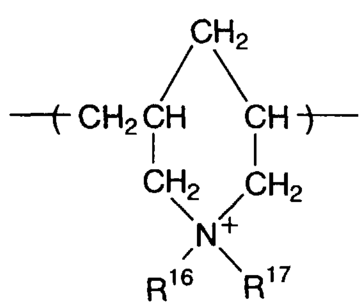

(4)

wherein $R^{16}$ and $R^{17}$ independently are a straight- or branched-chain alkyl group having 1 to 4 carbon atoms;

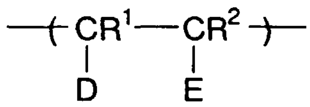

(5)

wherein $R^1$ and $R^2$ have the same meanings as defined above, D and E independently are a $-R^{18}COOM$, $-R^{18}SO_3M$ or $-R^{18}OPO_3M_2$ group (where M is a hydrogen atom or an alkali metal, $R^{18}$ is a simple bonding or an alkylene or hydroxyalkylene group having 1 to 4 carbon atoms), provided that when D and E are $-R^{18}COOM$ simultaneously, they may combine to form a cyclic anhydride group); or

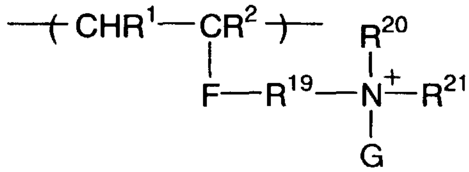

(6)

wherein $R^1$ and $R^2$ have the same meanings as defined above, F is an ether, ester or amide bonding, $R^{19}$ is an alkylene or hydroxyalkylene group having 1 to 4 carbon atoms, $R^{20}$ and $R^{21}$ independently are a methyl group or an ethyl group, G is a $-R^{22}COOM$, $-R^{22}SO_3M$ or $-R^{22}OPO_3M_2$ group (where M has the same meaning as defined above, $R^{22}$ is a simple bonding or an alkylene or hydroxyalkylene group having 1 to 4 carbon atoms).

4. The hair-care preparation as claimed in claim 3, wherein the polymer comprises a copolymer of 20 to 99.9% by weight of the repeating unit represented by general formula (1) described in claim 1 and 0.1 to 80% by weight of at least one of repeating units represented by general formula (7) and quaternary ammonium salts thereof :

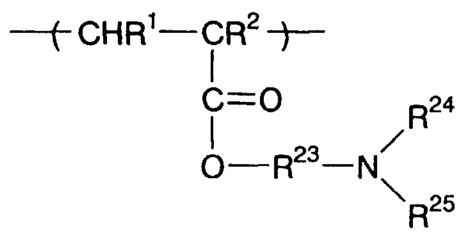

(7)

wherein $R^1$ and $R^2$ have the same meanings as defined above, $R^{23}$ is an alkylene or hydroxyalkylene group having 1 to 4 carbon atoms, $R^{24}$ and $R^{25}$ independently are a methyl group or an ethyl group.

5. The hair-care preparation as claimed in claim 3, wherein the polymer comprises a copolymer of 20 to 99.9% by weight of the repeating unit represented by general formula (1) described in claim 1 and 0.1 to 80% by weight of at least one of repeating units represented by general formula (8) and quaternary ammonium salts thereof :

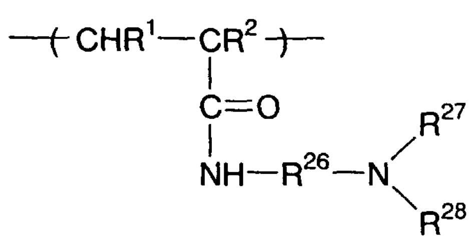

(8)

wherein $R^1$ and $R^2$ have the same meanings as defined above, $R^{26}$ is an alkylene or hydroxyalkylene group having

1 to 4 carbon atoms, $R^{27}$ and $R^{28}$ independently are a methyl group or an ethyl group.

**6.** The hair-care preparation as claimed in any of claims 1 to 5, wherein the polymer has a viscosity in 30% ethanol solution at 20°C as measured by Brookfield viscometer of 50 to 10,000 cps.

**7.** The hair-care preparation as claimed in any of claims 1 to 6, wherein the content of the polymer or copolymer is 0.01 to 40% by weight of the total composition.